# EUROPEAN PATENT APPLICATION

(11) **EP 4 464 714 A1**
(43) Date of publication of application: **20.11.2024**
(21) Application number: 23174274.3
(22) Date of filing: 19.05.2023
(51) Int. Cl.: C07K 7/06, C07K 7/08, G01N 33/68

(54) **SCREENING METHOD FOR CELL-REPELLING PEPTIDES**

(71) Applicant: Karlsruher Institut für Technologie, 76131 Karlsruhe (DE)
(72) Inventor: Nesterov-Müller, Alexander, 76661 Philippsburg (DE); Orian-Rousseau, Veronique, 67690 Rittershoffen (FR); Sonnentag, Steffen, Joachim, 76149 Karlsruhe (DE)
(74) Representative: Gille Hrabal Partnerschaftsgesellschaft mbB Patentanwälte

(57) **Abstract**

The invention relates to a method for screening for peptides, sequences and proteins with cell-repelling properties in a high-throughput screening method using peptide-chips and fluorescence detection. The invention further relates to peptides and sequences with cell-repelling properties, identified in the screening method of the invention, as well as to the use of such cell-repelling peptides and/or sequences.

## Description

### INTRODUCTION

The invention relates to a method for screening for peptides, sequences and proteins with cell-repelling properties in a high-throughput screening method using peptide-chips and fluorescence detection. The invention further relates to peptides and sequences with cell-repelling properties, identified in the screening method of the invention, as well as to the use of such cell-repelling peptides and/or sequences.

### BACKGROUND OF THE INVENTION AND PRIOR ART

The adhesion or repulsion of cells to the extracellular matrix engages cell surface receptors such as integrins, proteoglycans and other types of cell adhesion molecules such as CD44. For most cells, adhesion to a substrate is crucial for survival. Preventing normal cells from adhering will induce cell death, whereas anchorage-independent growth is a property of tumor cells. In normal tissues, epithelial cells are attached to a basement membrane that corresponds to a specialized part of the extracellular matrix (ECM). The ECM, composed of laminins, collagen, fibronectin, growth factors, glycosaminoglycans and other proteoglycans, controls the ability of cells to migrate, differentiate, survive or proliferate. Controlling cell adhesion or repulsion is therefore a means to control cell behavior. Accordingly, biomedical engineering and tissue engineering rely on the establishment of surfaces with adhesive and repellent properties.

The so far established way to study cell adhesion is to produce libraries of factors with activating or inhibiting properties that can be tested as coatings for cell attachment. To generate a large number of molecules at a high speed, this approach needs to be unbiased and efficient, as in the case of ribosomal or phage displays. The main challenge here is the synthesis of a large number of potential molecules and their transfer to cells.

Currently, there are different techniques for the implementation of such tasks. Most are based on 96-, 384- or 1536-well microplates (multiwell plates) with varying degrees of automation. High-throughput screening (HTS), however, offers high potential for automation but also has high costs that only pharmaceutical companies or a few research centers worldwide can afford. Over the past decade, several miniaturized platforms for cell-based assays have been proposed, such as the encapsulation of cells in droplets formed in an oil phase, SlipChip or droplet-array sandwiching technology. Miniaturized platforms are very efficient for cell phenotypic and transcriptomic analysis and significantly reduce reagent and cell consumption in comparison to current HTS. Using HeLa-CCL2 cells, it was shown that the same screening experiments as in HTS can be carried out with a significant reduction in culture volumes to 3 nL. However, volume miniaturization implies a change in cell cultivation conditions, such as pressure, diffusion parameters of the cells, and intensive accumulation of excretion products in the limited volume. In addition, cell culture for more than 24 hours in nL-scale compartments remains difficult due to evaporation issues and depletion of the cell culture medium.

Transfection on cell microarrays is an alternative approach that consists of pre-spotting transfection mixtures onto a glass slide prior to seeding of cells onto the slide. In this approach, cells take up the DNA or RNA on the printed areas, creating spots of localized transfection within a lawn of non-transfected cells. This type of screening made it possible to study cell transfection for many different DNA and RNA oligomer sequences (up to 10,000) without limiting the volume of the cell culture [Neumann, B. et al. Phenotypic profiling of the human genome by time-lapse microscopy reveals cell division genes. Nature 464, 721-727 (2010*)].*

Modern high-density peptide arrays synthesized in situ by the combinatorial deposition of amino acids can contain up to a million different peptide spots on a single substrate and thus represent an ideal pool of diverse functional molecules [Buus, S. et al. High-resolution Mapping of Linear Antibody Epitopes Using Ultrahigh- density Peptide Microarrays. Mol Cell Proteomics 11, 1790-1800 (2012*);* Legutki, J. B. et al. Scalable high-density peptide arrays for comprehensive health monitoring. Nat Commun 5 (2014*);* Jenne, F. et al. Resemblance-Ranking Peptide Library to Screen for Binders to Antibodies on a Peptidomic Scale. International Journal of Molecular Sciences 23 (2022*).*

This functionality can be substantially extended by the integration of artificial building blocks, peptide cyclization, or peptoid chemistry [Babii, O. et al. Diarylethene-Based Photoswitchable Inhibitors of Serine Proteases. Angew Chem Int Edit 60, 21789-21794 (2021*);* Roberts, K. D., Lambert, J. N., Ede, N. J. & Bray, A. M. Efficient synthesis of thioether-based cyclic peptide libraries. Tetrahedron Lett 39, 8357-8360 (1998*);* Streefkerk, D. E. et al. Synthesis of Constrained Tetracyclic Peptides by Consecutive CEPS, CLIPS, and Oxime Ligation. Org Lett 21, 2095-2100, doi:10.1021/acs.orglett.9b00378 (2019*);* Mattes, D. S. et al. Combinatorial Synthesis of Peptoid Arrays via Laser-Based Stacking of Multiple Polymer Nanolayers. Macromol Rapid Comm 40 (2019*)*]*.*

Different short peptides and peptidomimetics, such as Arg-Gly-Asp, either linear or constrained in a cyclic structure, have been generated and extensively studied for their capacity to regulate cell adhesion, migration, self-renewal, and pluripotency [Zhou, P. et al. Molecular basis for RGD-containing peptides supporting adhesion and self-renewal of human pluripotent stem cells on synthetic surface. Colloid Surface B 171, 451-460 (2018*);* Nicolas, J. et al. 3D Extracellular Matrix Mimics: Fundamental Concepts and Role of Materials Chemistry to Influence Stem Cell Fate. Biomacromolecules 21, 1968-1994 (2020*);* Kapp, T. G. et al. A Comprehensive Evaluation of the Activity and Selectivity Profile of Ligands for RGD-binding Integrins. Sci Rep-Uk 7 (2017*);* Sun, W. et al. Viability and neuronal differentiation of neural stem cells encapsulated in silk fibroin hydrogel functionalized with an IKVAV peptide. J Tissue Eng Regen M 11, 1532-1541 (2017*);* Jia, J. et al. Development of peptide-functionalized synthetic hydrogel microarrays for stem cell and tissue engineering applications. Acta Biomater 45, 110-120 (2016*)*].

Most of these peptides are derived from naturally occurring ECM macromolecules, which can be the source of new synthetic extracellular factors [Huettner, N., Dargaville, T. R. & Forget, A. Discovering Cell-Adhesion Peptides in Tissue Engineering: Beyond RGD. Trends Biotechnol 36, 372-383 (2018*);* Wang, F. et al. The Functions and Applications of RGD in Tumor Therapy and Tissue Engineering. International Journal of Molecular Sciences 14, 13447-13462 (2013*)].* Synthetic ECMs are particularly interesting in that their parameters, such as mechanical properties or permeability, can be more easily tuned.

Currently, mainly peptides with adherent properties are searched and investigated and in this respect, peptide arrays and cell compartmentation are often used. However, cell-arrays with separated compartments do not allow free flowing or movement of the cells in the system.

US2021/009782 and US2021/002450 describe methods for preparing peptide chips with selected peptides inducing cell adhesion via integrins, which are then used in methods of screening active pharmaceutical ingredients. That means, therein specific peptides are selected, which are already known for their cell-adhering properties and immobilized on a peptide-chip. No method for identifying the peptides themselves is described therein.

CD44v6 peptides have been described in context with suppressing metastasizing of tumor cells (AU2003238434), the inhibition of signaling via growth factors (EP1647556), as inhibitors of bacterial infections (US8933017), treating breast cancers (WO2014/079931), treating pancreatic cancer (WO2014/079940), and treating metastasizing cancers (WO2014/079943). Further, WO2014/079914 describes pegylated CD44v6-derived peptides.

Cell-repelling peptides have not been sufficiently studied so far, which may be due to the traditional extensive use of cell-repellent polymers such as poly(ethylene glycol) grafted-poly(L-lysine) 40 in cell patterning experiments. Another reason for this gap in cell-repellent peptide research may be due to the lack of high-throughput screening for such peptides. High-throughput screening methods for identifying and finding peptides having cell-repelling properties are not known or available so far.

### OBJECT OF THE INVENTION

It was the object of the invention to provide new methods for identifying and finding peptides having cell-repelling properties. In a further aspect the new method should provide high-throughput screening of peptides for such having cell-repelling properties. The new method should be easy to implement at reasonable costs and offer a high degree of automation to allow screening and analysis of a large number of different proteins, peptides and sequences. In particular, the new method should allow the screening for large amounts of different proteins, peptides or sequences in short time and simultaneously in one test setting. The new method should allow to be used with a broad variety of different cell types and in systems with high cell density, i.e. in heterogeneous cell cultures or in co-cultures, and should be broadly applicable and easy to implement in established cell culture systems. The new method should allow to be implemented in cell culture systems with large cell culture volumes to avoid the above-mentioned disadvantages resulting from miniaturization and working with small cell culture volumes. Concretely, the new method should be easy to implement in cell culture systems without disturbing or changing the cell cultivation conditions, such as pressure, diffusion parameters of the cells, or accumulation of excretion products in the limited volume. The new method should further be robust with respect to influences deriving from the cell cultures used. In a further aspect, the new method should provide additional benefits such as the possibility of evaluating cell behaviour in the communication with the tested proteins, peptides and sequences.

### SUMMARY OF THE INVENTION

To avoid the above-mentioned disadvantages of the prior art and solve the objects described above, the inventors of the present invention developed a new method for detecting and/or identifying peptides and/or sequences with cell-repelling properties. The new method is based on evaluating peptide-induced cell repulsion directly in cell culture systems via fluorescence detection using peptide-chips comprising attached to the chip surface a peptide and/or amino acid sequence library.

With the new method of the present invention the inventors developed a new possibility of screening for extracellular factors promoting the adhesion or detachment of cells using high-density peptide arrays without limiting the cell culture volume. The new method allows to use peptide arrays to conduct peptide mapping of peptides on proteins and to explore the functions of peptides at the level of individual amino acids. In a further aspect of the invention the inventors identified several sequences and peptides, with extreme cell repulsion properties.

The present invention includes, without being limited thereto, the following aspects:
[1] A method for detecting and/or identifying peptides and/or sequences with cell-repelling properties, which comprises:
(a) preparing or providing a chip which comprises attached to a chip-surface an array of multiple different peptides and/or sequences which are located adjacent to each other on the chip-surface to form a predetermined pattern of peptide- and/or sequence-spots;
(b) placing or providing the chip in a device suitable for receiving and/or culturing cells;
(c) adding cells and allowing the cells in the device to flow freely over the chip surface,
(d) fluorescence detection of the chip surface,
(e) detecting and/or identifying the peptides and/or sequences with cell-repelling properties via fluorescence signals.
[2] The method according to [1], further comprising a step (i) of microscopic evaluation of the chip surface.
[3] The method according to [1] or [2], wherein the peptides are attached to the chip-surface in the form of peptide- and/or sequence spots (pixels) having a size of ≤ 200 µm in diameter, preferably having a size of between 20 µm × 20 µm to 100 µm × 100 µm, preferably of 30 µm × 30 µm.
[4] The method according to [1] to [3], wherein the distance between the individual adjacent peptide- and/or sequence-spots (pixels) on the chip-surface is ≤ 1 pixel.
[5] The method according to [1] to [4], wherein the chip comprises ≥ 1000, preferably ≥ 3000, more preferably ≥ 5000, more preferably ≥ 10000 different peptides and/or sequences attached to the chip-surface.
[6] The method according to [1] to [5], wherein the different peptides / sequences are attached to the chip-surface with a spot density of > 20000 peptides / sequences per square centimetre.
[7] The method according to [1] to [6], wherein the device suitable for receiving and/or culturing cells is an incubation tray, a live cell imaging incubation chamber or a multiwell plate.
[8] The method according to [1] to [7], wherein the device suitable for receiving and/or culturing cells is capable to hold a cell culture volume of up to 10 ml of liquid culture medium.
[9] The method according to [1] to [8], which is carried out as high-throughput screening method.
[10] The method according to [1] to [9], wherein the detection and/or identification of the peptides and/or sequences with cell-repelling properties is carried out by continuous fluorescence detection or by fluorescence detection at a fixed endpoint after running the method for a predetermined time or by a combination of both.
[11] The method according to [1] to [10], wherein the fluorescence detection is carried out continuously over a period of up to 48 hours, preferably up to 36 hours, more preferably up to 24 hours, and/or at a fixed endpoint after at least 48 hours, preferably after at least 36 hours, more preferably after at least 24 hours of running the method.
[12] The method according to [11], wherein fluorescence detection is measured at repeating timepoints during running the method, preferably every 10 minutes.
[13] The method according to [1] to [12], wherein the peptides and/or sequences attached to the chip-surface are short-chain peptides with amino acid sequences of between 3 to 20 amino acids.
[14] The method according to [1] to [12], wherein the peptides attached to the chip-surface are medium- to long-chain peptides with amino acid sequences of more than 20 amino acids.
[15] The method according to [1] to [14], wherein the peptides attached to the chip-surface can be selected from linear peptides and/or sequences, cyclic peptides and/or sequences and from peptides and/or sequences comprising artificial (non-biogenic) amino acids, and combinations thereof.
[16] The method according to [1] to [15], wherein the peptides and/or sequences attached to the chip-surface are derived from known protein sequences by fragmentation and/or by substitution of one or more amino acids and/or by permutation of the protein sequences.
[17] The method according to [1] to [16], wherein the chip is designed to comprise at least one array of amino acid sequences which are fragments derived from one protein sequence by selecting multiple different fragments with identical chain length of consecutive parts of the protein sequence resulting in an array of amino acid sequence fragments having an overlap with respect to one, two or three amino acids of the protein sequence chain when comparing all fragments.
[18] The method according to [1] to [17], wherein the chip is designed to comprise at least one array of substituted amino acid sequences which are fragments derived from one protein sequence wherein in the fragments one amino acid of the originating protein sequence is replaced by one of the remaining nineteen biogenic amino acids.
[19] The method according to [1] to [18], wherein one to five copies of each peptide / sequence are attached to the chip surface.
[20] The method according to [1] to [19], wherein the peptides and/or sequences are attached to the chip-surface by immobilisation on a matrix covering a solid support or by direct synthesizing the peptides and/or sequences on the chip surface.
[21] Peptides and/or sequences with cell-repelling properties, according to the SEQ IDs No. 1 to 34:

| **SEQ IDNo** | **Peptide** |
|---|---|
| 1 | KQQ |
| 2 | PNATEASKP |
| 3 | EVK |
| 4 | AIHKWDKKN |
| 5 | SVLNSNAIK |
| 6 | VSAAP |
| 7 | TLSSKMYHTKGQ |
| 8 | WSKICKPVLKE |
| 9 | AEEALPKK |
| 10 | VRSSSRTP |
| 11 | SDKKPVAHVVANPQAE |
| 12 | YQTKV |
| 13 | PCQRETPEGAEAKP |
| 14 | CASEFALRMKIK**EVK** |
| 15 | ASEFALRMKIK**EVK**K |
| 16 | SEFALRMKIK**EVK**KE |
| 17 | EFALRMKIK**EVK**KEN |
| 18 | FALRMKIK**EVK**KENG |
| 19 | ALRMKIK**EVK**KENGD |
| 20 | LRMKIK**EVK**KENGDK |
| 21 | RMKIK**EVK**KENGDKK |
| 22 | MKIK**EVK**KENGDKKI |
| 23 | KIK**EVK**KENGDKKIV |
| 24 | IK**EVK**KENGDKKIVP |
| 25 | K**EVK**KENGDKKIVPK |
| 26 | **EVK**KENGDKKIVPKK |
| 27 | HPGSAVSASNAIKNL |
| 28 | TPPNATEASKPQGTT |
| 29 | AIKNLPPPTKGQEGS |
| 30 | QETWF |
| 31 | QNGNQGKN |
| 32 | IAMTPPNATEASKPQ |
| 33 | FLCSLFAPVCLDRPI |
| 34 | KENGDKKIVPKKKKP |

and homologues thereof having at least 70% identity, preferably at least 80% identity, more preferably at least 85% identity, even more preferably at least 90% identity, even more preferably at least 95% identity, and the sequences of SEQ ID 1 to 34, wherein one amino acid is replaced by one of the remaining nineteen biogenic amino acids.
[22] Use of peptides and/or sequences according to the SEQ IDs No. 1 to 37:

| **SEQ IDNo** | **Peptide** |
|---|---|
| 1 | KQQ |
| 2 | PNATEASKP |
| 3 | EVK |
| 4 | AIHKWDKKN |
| 5 | SVLNSNAIK |
| 6 | VSAAP |
| 7 | TLSSKMYHTKGQ |
| 8 | WSKICKPVLKE |
| 9 | AEEALPKK |
| 10 | VRSSSRTP |
| 11 | SDKKPVAHVVANPQAE |
| 12 | YQTKV |
| 13 | PCQRETPEGAEAKP |
| 14 | CASEFALRMKIK**EVK** |
| 15 | ASEFALRMKIK**EVK**K |
| 16 | SEFALRMKIK**EVK**KE |
| 17 | EFALRMKIK**EVK**KEN |
| 18 | FALRMKIK**EVK**KENG |
| 19 | ALRMKIK**EVK**KENGD |
| 20 | LRMKIK**EVK**KENGDK |
| 21 | RMKIK**EVK**KENGDKK |
| 22 | MKIK**EVK**KENGDKKI |
| 23 | KIK**EVK**KENGDKKIV |
| 24 | IK**EVK**KENGDKKIVP |
| 25 | K**EVK**KENGDKKIVPK |
| 26 | **EVK**KENGDKKIVPKK |
| 27 | HPGSAVSASNAIKNL |
| 28 | TPPNATEASKPQGTT |
| 29 | AIKNLPPPTKGQEGS |
| 30 | QETWF |
| 31 | QNGNQGKN |
| 32 | IAMTPPNATEASKPQ |
| 33 | FLCSLFAPVCLDRPI |
| 34 | KENGDKKIVPKKKKP |
| 35 | QETWFQNGWQGKN |
| 36 | QETWFQNGWQGKNP |
| 37 | KEQWFGNRWHEGYR |

and homologues thereof having at least 70% identity, preferably at least 80% identity, more preferably at least 85% identity, even more preferably at least 90% identity, even more preferably at least 95% identity, and the sequences of SEQ ID 1 to 37, wherein one amino acid is replaced by one of the remaining nineteen biogenic amino acids,
as cell-repelling peptides and/or sequences.
[23] Use of the peptides and/or sequences according to [22] for the modification of surfaces.
[24] The use according to [23] for the modification of surfaces in the medical, biomedical, biochemical field, tissue engineering.
[25] The use according to [23] or [24] for the modification of surfaces of medical or surgical devices, medical, biomedical, biochemical, diagnostic or tissue engineering equipment.

The present invention is described in more detail as follows.

### DETAILED DESCRIPTION OF THE INVENTION

The new method of the invention is based on the principle of attaching the peptides or amino acid sequences to be evaluated onto a chip surface (carrier or substrate) to provide a peptide chip with an array of peptides and sequences attached thereto and to introduce said peptide chip into a cell culture system which allows free flowing and movement of the cells over the chip surface and detecting the repulsion of the cells on the peptide chip via fluorescence detection and therewith identify those peptides or sequences, which repel the cells.

In the method of the invention for detecting and/or identifying peptides and/or sequences with cell-repelling properties a so-called peptide chip is used. In principle, peptide chips and the method for preparing such chips are well known to a person skilled in the art. Therein, a multitude of peptides and/or amino acid sequences are attached or printed onto a chip surface, i.e. onto a substrate or carrier material. Methods of attaching or printing of the peptides and/or sequences onto the chip-surface comprise immobilisation thereof on a matrix covering a solid support, such as e.g. an inert carrier material (e.g. glas or plastic) covered with a suitable polymer layer e.g. such as described in the prior art above (US2021/009782 and US2021/002450). It is also possible to attach the peptides / sequences onto the chip surface by direct synthesizing the peptides and/or sequences from the amino acids on the chip surface with methods well known in the art. In the case of direct synthesis of peptides, different amino acid derivatives both, canonical as well as non-canonical, are addressed to different spots onto the chip surface using the protection group strategies as developed in the scope of the solid phase peptide synthesis. The addressing of synthesis monomers can be defined mechanically with coordinates printer nozzles, electrically by generating electric field patterns or optically by applying irradiation patterns.

Regarding the description of methods for preparing the peptide-chips of the present invention reference is made to established methods, e.g. such methods as described in detail by Jenne, F.; Biniaminov, S.; Biniaminov, N.; Marquardt, P.; Von Bojnicic-Kninski, C.; Popov, R.; Seckinger, A.; Hose, D.; Nesterov-Mueller, A. Resemblance-Ranking Peptide Library to Screen for Binders to Antibodies on a Peptidomic Scale. Int. J. Mol. Sci. 2022, 23, 3515*.;* or by Fodor, S. P.; Read, J.L.; Pirrung, M.C.; Stryer, L.; Lu, A. T.; Solas, D. Light-directed, spatially addressable parallel chemical synthesis. Science 1991, 251, 767-773*.;* or by Loeffler, F.F.; Foertsch, T.C.; Popov, R.; Mattes, D.S.; Schlageter, M.; Sedlmayr, M.; Ridder, B.; Dang, F.X.; von Bojnicic-Kninski, C.; Weber, L.K.; et al. High-flexibility combinatorial peptide synthesis with laser-based transfer of monomers in solid matrix material. Nat. Commun. 2016, 7, 11844*.;* or in Life 2023, 13, 796 14 of 15*;* or by Paris, G.; Heidepriem, J.; Tsouka, A.; Liu, Y.; Mattes, D.S.; Pinzon Martin, S.; Dallabernardina, P.; Mende, M.; Lindner, C.; Wawrzinek, R.; et al. Automated Laser-Transfer Synthesis of High-Density Microarrays for Infectious Disease Screening. Adv.Mater. 2022, 34, e2200359*.*

Generally, such a peptide chip comprises attached to the chip surface multiple peptides and/or amino acid sequences in the form of small spots or pixels, which are located adjacent to each other on the chip-surface in a predetermined array. By attaching multiple different peptides and/or sequences, a so-called peptide library can be provided on such a chip and tested in the test setting. The peptide library on a peptide chip can be designed to form a predetermined pattern of peptide- and/or sequence-spots. Such pattern or array of peptides / sequences provides a systematic arrangement of the peptides / sequences, usually in rows and columns, to determine the specific position of every individual peptide / sequence on the chip. This allows to clearly identify, which peptide / sequence on which chip position repels the cells, when analysing the chip surface after cell incubation via fluorescence detection. It is also possible to use specific templates to divide a chip for applying different cell cultures, culture media or cultivation/incubation conditions on one chip.

The selection of peptides and sequences attached to the chip surface can be arbitrary or follow certain fragmentation patterns, e.g. by attaching consecutive fragments with overlapping sequences derived from a target protein of particular interest. It is also possible to attach peptides / sequences following a systematic substitution pattern. Such peptide / sequence arrays of substituted amino acid sequences are preferably derived by using one originating protein sequence and similar sequences, wherein systematically one amino acid is replaced by one of the remaining nineteen biogenic amino acids. It is also possible to use cytokines, e.g. cytokines known from sequencing. The peptides and sequences attached to the chip surface can be linear peptides and/or sequences, cyclic peptides and/or sequences and peptides and/or sequences comprising artificial (non-biogenic) amino acids. The chip used in the method of the present invention may further comprise peptides and amino acid sequences, which are derived by permutation of the protein sequences.

In the method of the invention the peptides and/or sequences attached to the chip-surface are preferably short-chain peptides with amino acid sequences of between 3 to 20 amino acids. However, it is also possible to use chips wherein the peptides attached to the chip-surface are medium- to long-chain peptides with amino acid sequences of more than 20 amino acids.

In one aspect of the invention the peptides and/or sequences attached to the chip-surface are derived from known protein sequences by fragmentation, e.g. by cutting the protein sequence at multiple different points of the chain into fragments of between 3 to 20 amino acids, preferably 3 to 16 amino acids, which therewith represent different consecutive sections of the originating protein sequence.

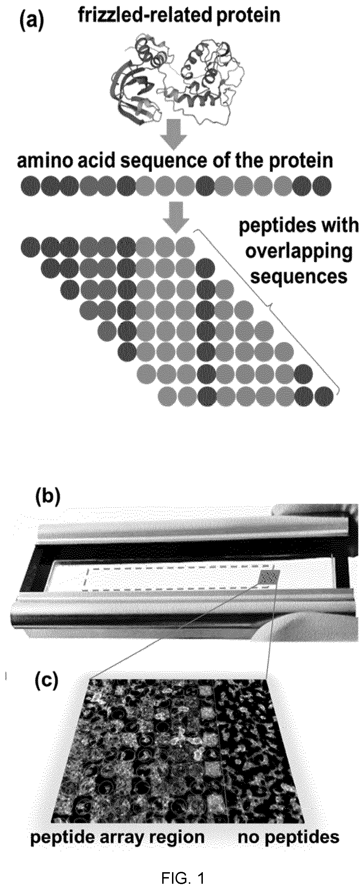

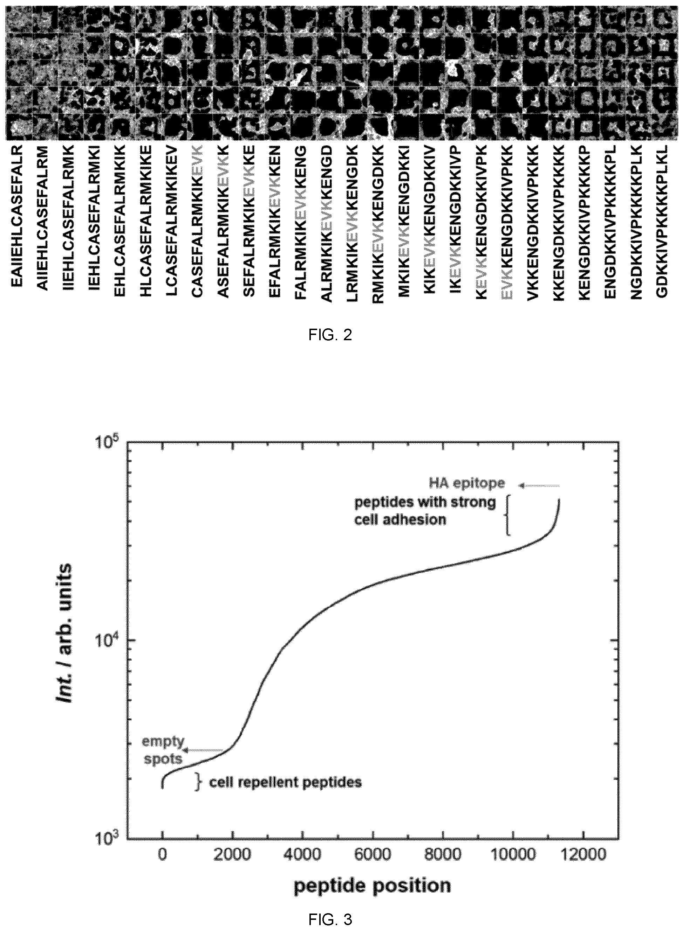

In one specific aspect of the invention the chip is designed to comprise at least one array of amino acid sequences which are fragments derived from one (originating) protein sequence by selecting multiple different fragments, each having the same chain length (same amount of amino acids), and which represent different consecutive sections of the originating protein sequence. Such array represents an array of amino acid sequence fragments, wherein all fragments have an overlap with respect to one, two or three (consecutive) amino acids of the originating protein sequence chain, preferably an overlap of three consecutive amino acids, when comparing all fragments with each other. This means, the position of the overlapping (preferably three) amino acid motif of the originating protein sequence, which is present in all fragments in the array, so to say moves within the fragments deriving from the originating protein sequence. Such a moving sequence motif position is illustrated in Figures 1(a) and 2 below.

In a further aspect of the invention the chip is designed to comprise at least one array of substituted amino acid sequences which are fragments derived from one (originating) protein sequence wherein in the fragments one amino acid of the originating protein sequence is replaced by one of the remaining nineteen biogenic amino acids.

The peptides are attached to the chip-surface in the form of peptide- and/or sequence spots, also called "pixels". Such "pixels" may have different geometric forms such as circles, squares etc. but may also have no exact and specific geometric form. Such spots or pixels preferably have a characteristic size of ≤ 200 µm, more preferably ≤ 100 µm in diameter. The characteristic size of the pixels is preferably between 20 µm and 100 µm, preferably of about 30 µm in diameter. A diameter size defined for pixels not having an exact round geometric form, e.g. a square form, relates to an approximate diameter of the geometric form, measured between two most distant opposite points. In a further aspect, the spots or pixels of the peptide chips have a size of between 10 µm × 10 µm, 20 µm × 20 µm, 30 µm × 30 µm, 40 µm × 40 µm, 50 µm × 50 µm, up to 100 µm × 100 µm.

The distance between two individual adjacent peptide- and/or sequence-spots (pixels) on the chip-surface is ≤ 3 pixel, preferably ≤ 2 pixel, more preferably ≤ 1 pixel. The selection of the distance between adjacent spots may vary depending on the size and form of the cells evaluated and/or on the number of spots (peptides) to be attached onto the chip surface. With narrower spots more peptides can be attached on a surface of predetermined size. If the spots are too narrow to each other, adherence / repelling behaviour of cells on adjacent peptide spots may become difficult to detect clearly.

The size and geometry of the peptide chip is not particularly limited and can be varied depending on the attached number of peptides or sequences and/or the size of the spots. The size and geometry of the chips can further be varied with respect to the cell culture test setting into which they are introduced. In one aspect of the invention, the peptide chips can be provided in the form of single, individual chips, which can be inserted into the desired cell culture setting (e.g. a petri dish or cell culture flask etc.). In another aspect of the invention the peptide chips can be prepared in the form of attaching (printing) the peptides and sequences onto a substrate, carrier or matrix or directly onto the bottom or walls of test wells, e.g. 96-, 384- or 1536-well microplates.

Generally, to allow testing of large amounts of peptides and sequences in efficient time, chips with ≥ 1000 peptides are used. Preferably, chips comprising ≥ 3000, more preferably ≥ 5000, even more preferably ≥ 10000 peptides and/or sequences are used.

The spot density on the chip-surface can vary depending on the conditions discussed above. In one aspect of the invention, the spot density on the chip surface amounts to > 20000 peptides and/or sequences per square centimetre.

Generally, the chip used in the method of the present invention comprises different peptides and/or sequences to provide a peptide library to be scanned for cell repelling peptides. However, it is possible to provide 1, 2, 3, 4 or 5 copies of one or of several or of all of the peptides and/or sequences attached to the chip surface.

In principle, the device suitable for receiving and/or culturing cells can be any suitable cell culture device, including petri dishes, cell culture flasks, tubes as well as multiwell plates. In one aspect of the invention the device suitable for receiving and/or culturing cells is an incubation tray or a live cell imaging incubation chamber. In the cell culture device used in the method of the invention the cells added or cultured therein shall be able to freely flow and move over the chip surface. Preferably, the cells are cultured and incubated in the cell culture device with the peptide chip placed or provided therein, usually on the bottom of said device.

Preferably, the device suitable for receiving and/or culturing cells is capable to hold a cell culture volume needed for providing a stable cell culture system. Examples of cell culture volumes, which should be able to implement amount up to 10 ml or at least up to 5 ml of liquid culture medium. The volume can be varied depending on the cell culture, cell culture conditions (CO₂, temperature), test setting, cell culture device and chip size. Smaller chips can be placed in devices with small cell culture medium volumes, when using larger chips, the devices and therewith the cell culture medium volumes will increase. The dimensions of the cell culture device and cell culture medium can be adapted depending on the specific test setting and needs, as long as free movement and flowing of the cells over the chip surface in the device and the detection of repulsion behaviour is possible.

In principle all kinds of cells can be used, including for example stem cells, cancer cells, epithelial cells, primary cells but also genetically modified organisms (GMO) and bacteria. It is also possible to cultivate three dimensional structures, such as spheroids. Homogenous as well as heterogeneous cell cultures can be used as well as co-cultures.

Generally, after combining the cells and the peptide chip in the setting of the method of the invention, the cells are incubated for a certain time on the surface of the peptide chip. Such incubation may be carried out from 10 minutes up to several days, depending on the cell culture, cell type or other conditions. Preferably, the incubation is carried out for some hours, such as 3, 4, 5, 6, 7 and up to 12 hours. In a preferred aspect the incubation is carried out for not longer than 24 hours. Usually, these time periods are sufficient to observe cell repulsion behaviour and usually, cell adhesion / repulsion of the cells is sufficiently completed to allow identification of repelling peptides at the latest after 24 hours. If no clear identification occurs after 24 hours, the incubation and measurement can be prolonged. End-point determination can be monitored via continuous and/or intermitting fluorescence detection.

During the incubation the cells interact with the peptides / sequences on the chip surface and depending on adhering or repelling properties of the peptides / sequences, the cells move to the spots where adhering is possible. This leads to the formation of cell patterns on the chip corresponding to the repelling effect of the peptides/sequences.

Via such formation of cell patterns the repelling properties of the peptides and sequences on the chip surface can be visualized and determined optically using fluorescence detection. The fluorescence detection visualizes on which spots no cells adhere in the incubation period and therewith allows to identify the cell-repelling peptides / sequences via their specific position in the chip array. Such identification and peptide determination in the array can be conducted automatically with suitable computer implemented readers or detection units.

As mentioned above, for identifying those peptides / sequences with repelling properties, the fluorescence detection can be carried out at a certain (fixed) end-point, e.g. after the incubation period. In such cases the fixed endpoint is preferably set after at least 48 hours, preferably after at least 36 hours, more preferably after at least 24 hours.

As mentioned above, it is also possible to carry out fluorescence detection continuously over a predetermined period of time, e.g. over the whole or a part of the incubation period. In such cases the fluorescence detection is preferably carried out continuously over a period of up to 48 hours, preferably up to 36 hours, more preferably up to 24 hours. Continuous fluorescence detection may also be of interest for monitoring the cell behaviour during the incubation period and during the adhering / repulsion process while moving over the chip surface. The specific movement of the cells may provide valuable information for their characterization.

As mentioned above, it is further possible to carry out fluorescence detection at repeating timepoints or in intervals during running the method, such as e.g. every 5, 10, 15, 30 or 60 minutes until the adhering / repelling process of the cells is completed an no changes in the cell movements can be detected any more. This may help to determine a suitable fixed measurement endpoint.

In principle, continuous, intermitting and end-point fluorescence detection can be combined.

Preferably, the whole chip surface is detected, but in any case, at least those parts of the chip surface are detected onto which peptides / sequences are attached in the test setting. Fluorescence detection can be carried out with conventional fluorescence detection devices, preferably with automated evaluation units.

The method of the invention can combine further detection methods, such as in particular microscopic evaluation of the chip surface. This allows to evaluate additional aspects of the cell cultures, e.g. cell communication with each other and with the surface, stress reactions of the cells etc., and therewith allows to provide additional valuable information for cell characterization.

A particular advantage of the new method of the invention can be seen in that it can be carried out as a high-throughput screening method.

With the method of the invention as described herein, the inventors identified the following peptides and/or sequences to have cell-repelling properties:

**Table 1**

| **SEQ ID No.** | **(originating) Protein** | **Peptide / Sequence** |
|---|---|---|
| 1 | SFRP1 | KQQ |
| 2 | SFRP1 | PNATEASKP |
| 3 | SFRP1 | EVK |
| 4 | SFRP1 | AIHKWDKKN |
| 5 | DKK1 | SVLNSNAIK |
| 6 | DKK1 | VSAAP |
| 7 | DKK1 | TLSSKMYHTKGQ |
| 8 | DKK1 | WSKICKPVLKE |
| 9 | TNF-alpha | AEEALPKK |
| 10 | TNF-alpha | VRSSSRTP |
| 11 | TNF-alpha | SDKKPVAHVVANPQAE |
| 12 | TNF-alpha | YQTKV |
| 13 | TNF-alpha | PCQRETPEGAEAKP |
| 14 | | CASEFALRMKIK**EVK** |
| 15 | | ASEFALRMKIK**EVK**K |
| 16 | | SEFALRMKIK**EVK**KE |
| 17 | | EFALRMKIK**EVK**KEN |
| 18 | | FALRMKIK**EVK**KENG |
| 19 | | ALRMKIK**EVK**KENGD |
| 20 | | LRMKIK**EVK**KENGDK |
| 21 | | RMKIK**EVK**KENGDKK |
| 22 | | MKIK**EVK**KENGDKKI |
| 23 | | KIK**EVK**KENGDKKIV |
| 24 | | IK**EVK**KENGDKKIVP |
| 25 | | K**EVK**KENGDKKIVPK |
| 26 | | **EVK**KENGDKKIVPKK |

Further peptides and sequences identified and used in the Examples are the following:

**Table 2**

| **Further Sequences from the Examples** | | |
|---|---|---|
| **SEQ ID No.** | **(originating) Protein** | **Peptide / Sequence** |
| 27 | *DKK1* / *SFRP1* | HPGSAVSASNAIKNL |
| 28 | *DKK1* / *SFRP1* | TPPNATEASKPQGTT |
| 29 | *DKK1* / *SFRP1* | AIKNLPPPTKGQEGS |
| 30 | *DKK1* / *SFRP1* | QETWF |
| 31 | | QNGNQGKN |
| 32 | | IAMTPPNATEASKPQ |
| 33 | | FLCSLFAPVCLDRPI |
| 34 | | KENGDKKIVPKKKKP |
| 35 | | QETWFQNGWQGKN |
| 36 | | QETWFQNGWQGKNP |
| 37 | | KEQWFGNRWHEGYR |

Such peptides / sequences constitute a further aspect of the invention. The invention further comprises homologues of the peptides / sequences shown above (i.e. those with SEQ IDs 1 to 37) having at least 70% identity, preferably at least 80% identity, more preferably at least 85% identity, even more preferably at least 90% identity, even more preferably at least 95% identity, even more preferably at least 99% identity, with the amino acid sequences SEQ ID No. 1 to 37 as a whole. In the context of the present invention, identity percentages for amino acid sequences are determined / calculated using the Software-Tool Blastp (Protein BLAST: search protein databases using a protein query (nih.gov); Altschul, S.F., Gish, W, Miller, W., Myers, E.W., Lipman, D.J. (1990) "Basic local alignment search tool." J. Mol. Biol. 215:403-410*).*

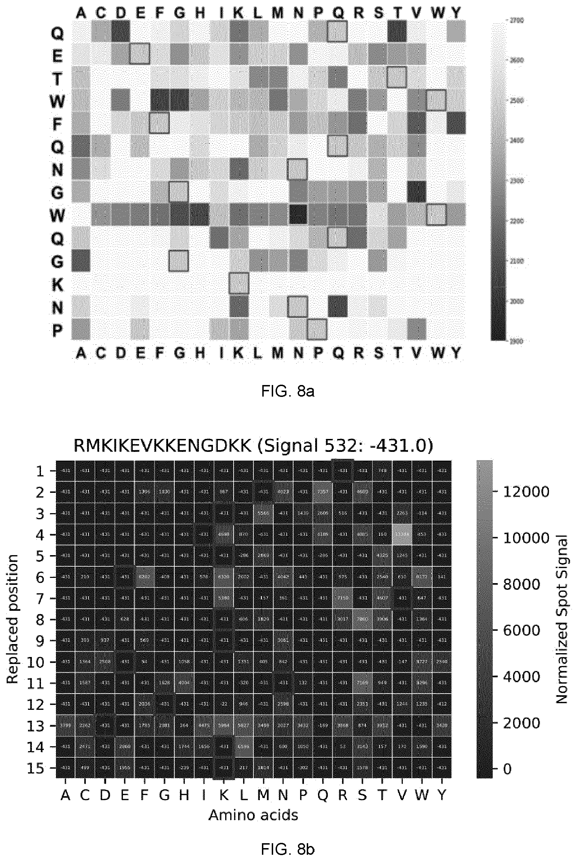

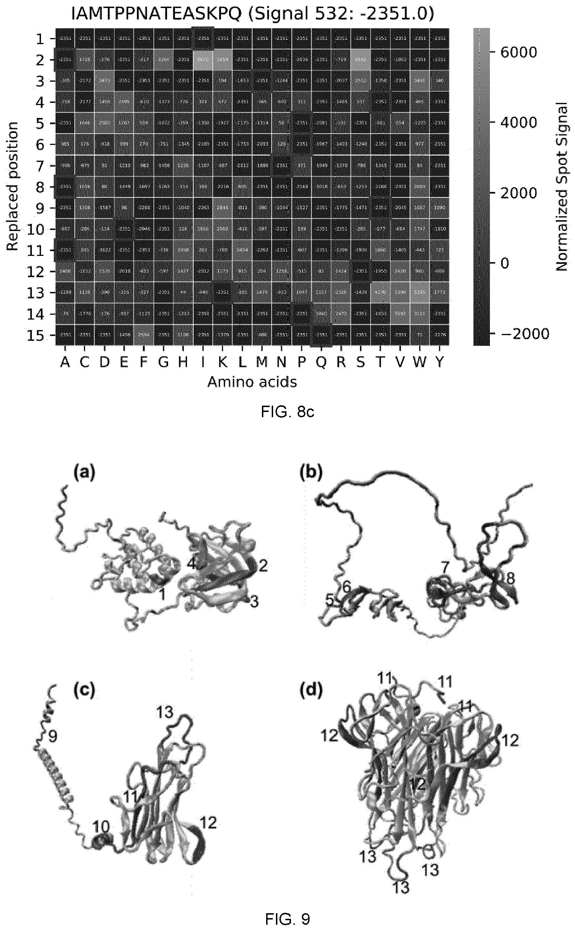

The invention further comprises the peptides / sequences shown above (i.e. those with SEQ IDs 1 to 37), wherein one amino acid is replaced by one of the remaining nineteen biogenic amino acids. Examples of substitution patterns are shown in Fig. 8a, 8b and 8c. Fig. 8a shows a substitution pattern of a peptide with the SEQ ID No. 35, Fig. 8b shows a substitution pattern of a peptide with the SEQ ID No. 21, while Fig. 8c shows a substitution pattern of a peptide with the SEQ ID No. 32.

In a preferred aspect the invention relates to one or more of the exact peptides / sequences with the SEQ IDs 1 to 34 as listed in Table 1 and/or 2 or one or more of the sequences from the substitution patterns shown in Fig. 8a), 8b) and 8c).

A further aspect of the invention relates to the use of the peptides and/or sequences shown above (i.e. those with SEQ IDs 1 to 37) and their above defined homologues and substituted amino acid sequences, e.g. those shown in the substitution patterns shown in Fig. 8a), 8b) and 8c), as cell-repelling peptides and/or sequences.

In the above sequences with SEQ ID No. 14 to 26 the inventors identified the three amino acids motif EVK as a common motif in cell repelling peptides.

The cell-repelling peptides and/or sequences identified with the method of the present invention and as described herein can, in a particular aspect of the invention, be used for the modification of surfaces to equip a surface with cell repelling properties. Such cell repelling surface modification equipment is of particular interest in the medical, biomedical, biochemical field and in tissue engineering applications. For example, medical, diagnostic or surgical devices used in the medical or diagnostic field, including surgery devices or implants, may benefit from specific cell repelling properties as well as medical, biomedical, biochemical, diagnostic or tissue engineering equipment.

Generally, the method of the invention can be used widely in research and development, including the medical field in general, biomedicine, medical technology, cell biology, drug development and testing, pharmacology and toxicity, organic chemistry, microsystems technology etc.. The new method offers the possibility to evaluate and identify potential compound candidates for the desired application field fast and with high throughput. Examples of potential products, which can be developed using the method of the invention, include artificial cell messenger substances, inhibitors of signal paths of cells based on cell-adhering and cell-repelling peptides, cell factors for the characterisation of cell types, cell factors for microstructure set-ups for research and development and/or implementation of devices using cell patterns.

The invention is illustrated further in the Examples and Figures below.

### DESCRIPTION OF THE FIGURES

- Fig. 1: Setup and principle of the high-throughput screening method of the invention using a high-density peptide array.
(a) Design of an exemplary peptide array with consecutive fragments of a functional protein with one overlapping amino acid
(b) Incubation well of a tray with schematically shown region of the peptide array (dashed rectangle).
(c) Fluorescence image of an exemplary peptide array with fluorescently labelled cells with peptide pixels exhibiting strong cell repulsion visualized via black pixels in the peptide array.
- Fig. 2: Peptide mapping of a selected region of the frizzled-related protein 1-SFRP1 after incubation with fluorescently labelled cancer cells on a peptide chip comprising five copies of each peptide arranged in a column and visualizing cell deposition with good adherence (left), repulsion (middle), or island-like patterns (right) in an array comprising fragments with a moving overlapping three amino acids motif (EVK) in the fragments attached to the chip.
- Fig. 3: Intensity measurement of the fluorescence signals from cells on peptide spots, arranged in ascending order for the entire peptide library with arrows marking the signal from empty spots and from a control HA epitope and with curly brackets at the regions of the curve's maximum gradients indicating the regions of peptides with specific cell adhesion or attraction.

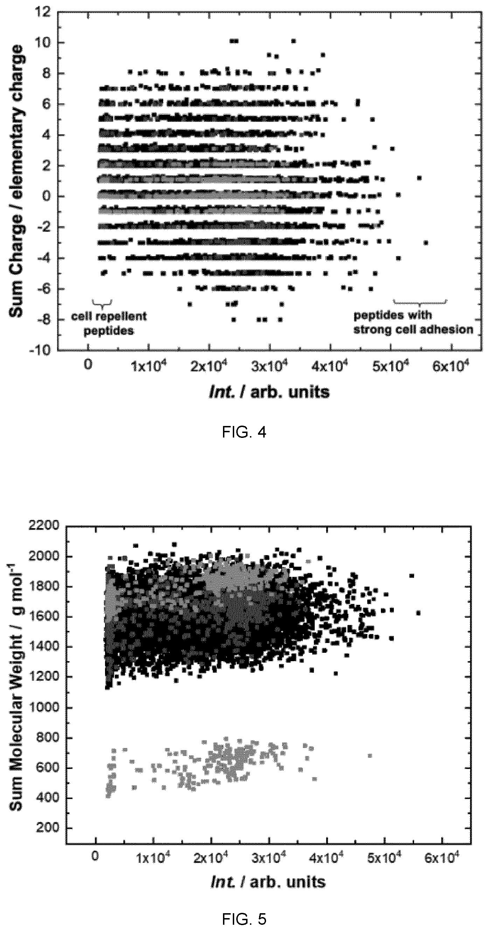

- Fig. 4: Scatter plot: fluorescence intensity; Int. versus the sum of charges (definition according to Moore, D. S. Amino-Acid and Peptide Net Charges - a Simple Calculational Procedure. Biochem Educ 13, 10-11 (1985*)*) for each peptide with the shaded dots indicating the peptides from the three different groups: overlapping peptides to map proteins, substitutions of special peptides and random peptides.
- Fig. 5: Scatter plot: fluorescence intensity; Int. versus the sum of molecular weight with a separately arranged cluster of low molecular weight dots (at the bottom part) corresponding to 5-mer peptides from the substitutional library and a large cluster (at the top part) consisting of 15-mer and 14-mer peptides (shaded dots) from all free peptide groups.

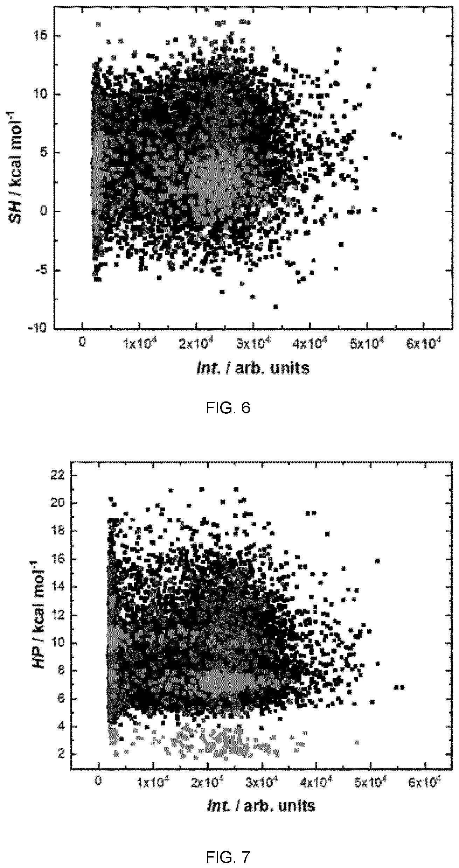

- Fig. 6: Scatter plot: fluorescence intensity; Int. versus the sum of hydrophobicity (SH).
- Fig. 7: Scatter plot: fluorescence intensity; Int. versus the sum of helix propensity (HP).
- Fig. 8: (a) Schematic illustration of a substitution pattern for the peptide QETWFQNGWQGKN (SEQ ID No. 35) with square marks indicating the substitution positions and showing that replacement of the amino acid W by N in an accordingly modified peptide QETWFQNGNQGKN exhibits maximum cell repulsion;
(b) illustration of a substitution pattern for the peptide RMKIKEVKKENGDKK (SEQ ID No. 21) with square marks indicating the substitution positions;
(c) illustration of a substitution pattern for the peptide IAMTPPNATEASKPQ (SEQ ID No. 32) with square marks indicating the substitution positions.
- Fig. 9: Mapping of cell-repellent peptides on 3D protein models, wherein cell-repellent peptides, identified with the method of the invention, are marked.
(a) Secreted frizzled-related protein 1.
(b) Dickkopf-related protein 1.
(c) Tumour necrosis factor.
(d) Tumour necrosis factor as a homotrimer, with the peptides with repulsive properties clearly expressed at the flexible poles (positions 11 and 13) of the TNF-alpha homotrimer and the numbers representing the SEQ ID No. of peptides according to Table 1.

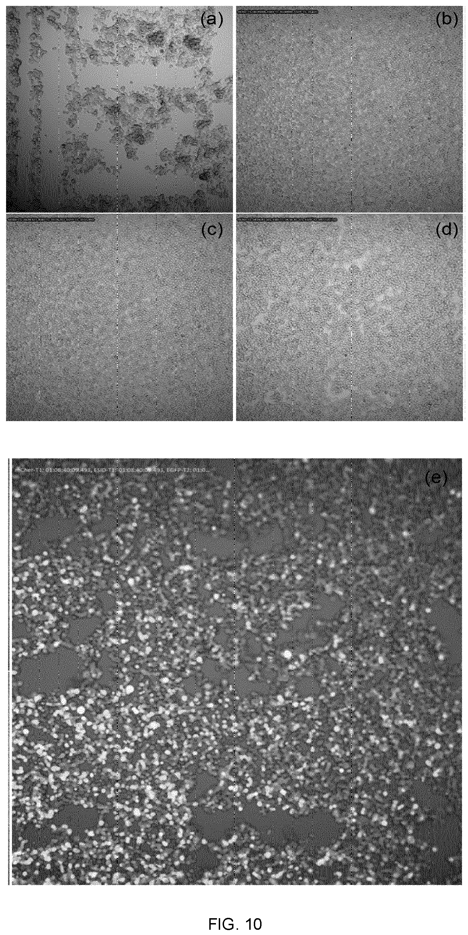

- Fig. 10: Development of cells on patterns of cell-repellent peptides with pictures deriving from continuous fluorescence detection via video recording made with a confocal microscope.
(a) Cell pattern on the chip surface in the form of the letters "KI", formed by cell-repelling peptides, with a size of 3 synthesis pixels (spots) and two lines with sizes of 3 synthesis pixels (spots) and 1 synthesis pixel (spot), each spot having a size of 30 µm in diameter, at the end of the experiment (after 24 hr of incubation).
(b) Chip surface at the beginning of the experiment (0 hrs 0 min).
(c) Chip surface after 10 min of incubation.
(d) Cell pattern on the chip surface after 5 hr of incubation.
(e) Cell pattern on the chip surface after 24 hr of incubation.

### EXAMPLES

The invention is described further by the following examples, without being limited thereto.

### Chip Design and Experimental Setup

A peptide chip was prepared, comprising in total a peptide library of 11,314 unique fragments with three groups (arrays) deriving from three originating proteins, namely
(i) secreted frizzled-related protein (SFRP1, Q8N474),
(ii) Dickkopf-related protein (DKK1, O94907), and
(iii) tumour necrosis factor (TNF-alpha, Q5STB3)

These originating proteins were taken from the UniProt database [MacDougall, A. et al. UniRule: a unified rule resource for automatic annotation in the UniProt Knowledgebase (vol 36, pg 4643, 2020*);* Bioinformatics 36, 5562-5562 (2020*)*]

The first group comprised 771 15-mer amino acid sequences derived from protein sequences with an overlap of one amino acid (Fig. 1a). Five copies of each peptide were attached onto the chip.

The second group comprised 726 substitution sequences derived from four peptides: 5-mer NRWHE (1) and NGWQG (2) and 14-mers KEQWFGNRWHEGYR (1) and QETWFQNGWQGKNP (2), which have been reported to inhibit the co-receptor function of CD44v6 of the receptor tyrosine kinase MET in a human (1) or murine (2) background and thereby inhibit pancreatic tumour growth and metastasis [Matzke-Ogi, A. et al. Inhibition of Tumor Growth and Metastasis in Pancreatic Cancer Models by Interference With CD44v6 Signaling. Gastroenterology 150, 513-525 e510, doi: 10. 10531jgastro.2015.10.020 (2016*)].* Substitutions corresponded to a replacement of one amino acid at each position with the remaining 19 biogenic amino acids. Three copies of each peptide from the second group were plated.

The third group of peptides was represented by 9818 fifteen-mer sequences based on random combinations of peptide fragments from the first group. Two copies of each peptide from the third group were plated.

In addition, blank spots and human influenza haemagglutinin HA-epitope (YPYDVPDYA) spots were placed on the chip as controls. HA-epitope was used to control the quality of the peptide chip by incubating it with labelled anti-HA antibodies [Konig, K. et al. Programmable high voltage CMOS chips for particle-based high-density combinatorial peptide synthesis. Sensor Actuat B-Chem 147, 418-427 (2010*).]*

The peptide library was synthetized via the high-density peptide microarray technology of axxelera 32 (Karlsruhe, Germany). The minimum synthesis area, a synthesis pixel (s-pixel), was 30 µm × 30 µm. The peptides and their copies were arranged randomly on the peptide chip to prevent local effects on cell adhesion. Next, the chip was placed either in an incubation tray (Fig. 1b) or in a live cell imaging incubation chamber. Approximately 10 7 SW620 colorectal cancer cells expressing the TOP-GFP construct, reflecting the activity of the Wnt signalling pathway by the expression of GFP, in 3 ml of liquid culture medium were transferred onto the chip and incubated for 24 hours.

After incubation, the chips were analysed using the confocal fluorescence scanner Innoscan 1100 AL (Innopsys, Carbonne, France). A wide range of diverse cellular patterns were observed, from peptide spots with densely packed cells to spots with no cells at all (Fig. 1c).

### Cellular Patterns

Fig. 2 shows changes in cell adhesion for peptides with an overlap of a three amino acids motif moving in the sequences of the consecutive fragments spanning the entire protein sequence. In this way, the EVK motif was identified as a repellent motif for cells. A large number of cell patterns exhibited incomplete filling of peptide spots. Some show a well-defined island shape, such as the peptide KENGDKKIVPKKKKP.

Fig. 3 provides an overview of how the entire peptide library affected cell adhesion. The graph shows cell adhesion, measured by the strength of the fluorescent signal, for all peptides arranged in ascending order. While the signal increase in most parts of the curve is smooth, a significant increase in the intensity gradient occurs at the edges of the curve. These two sites of the curve correspond to peptides with extreme repulsion or attraction of cells.

To understand how the sequence influences the adhesion or cell repulsion properties of the peptides, scatter plots reflecting various characteristics of peptides were prepared, wherein the sum of charges (total charges), the sum of molecular weights (total molecular weights), the sum of hydrophobicity (total hydrophobicity) and the sum of helix forming propensity (total helix forming propensity) were calculated (Fig. 4, 5, 6 and 7). Some of the randomly combined peptides from the third group (black dots) possessed maximum adhesion properties compared to the peptides from the first and second groups (shaded dots).

This strong adhesion was not correlated with either the total charge (Fig. 4) or the total molecular weight (Fig. 5). The independence of the adhesion and the total molecular weight was especially clear in the example of peptides from the second group (bright dots), where peptides with different lengths and thus different molecular weights demonstrated the same adhesion (Fig. 5). However, there was a weak trend towards greater total hydrophobicity (Fig. 6) and lower total helix forming propensity (Fig. 7) for peptides with strong adhesive properties. The control HA epitope was the peptide with the highest adhesion to cells.

In the region of amino acid sequences with strong cell repulsion (fluorescence intensity tends to zero), the wide distribution of dots over the total hydrophobicity or total helix forming propensity reflects the lack of correlation between the repulsion effect and these properties of the peptides. Thus, it was necessary to consider each sequence separately.

### Strong Cell Repulsion Peptides

The first three peptides with the strongest cell-repellent properties were
HPGSAVSASNAIKNL,
TPPNATEASKPQGTT, and
AIKNLPPPTKGQEGS
which are fragments of the DKK1 and SFRP1 proteins. Other peptides of these proteins also appeared in the top ten cell-repellent peptides, as well as peptide QETWF.

QNGNQGKN from the second group of the peptide library is a single mutation of the CD44v6-inhibiting peptide QETWFQNGWQGKN, where the amino acid W was replaced by N. In this case, the position of this mutation is important, since the appearance of amino acid N in other positions does not lead to strong cell repulsion (Fig. 8a)). Random combinatorial peptides from the third group, despite their significant numerical superiority in the library, were clearly underrepresented in the group of the strongest cell-repellent peptides.

Further, Fig. 8b) and Fig. 8c) show substitution patterns with the sequences RMKIKEVKKENGDKK (SEQ ID 21) and IAMTPPNATEASKPQ (SEQ ID 32), respectively.

The peptides with the SEQ ID Nos. 1 to 13 according to Table 1 were mapped to the 3D structures of the corresponding proteins, which were predicted using the Al-based tool AlphaFold [Jumper, J. et al. Highly accurate protein structure prediction with AlphaFold. Nature 596, 583-+ (2021*)*] (Fig. 9a-c). The first feature of this mapping is that peptides with strong cell repulsion are not associated with a specific secondary structure. They could be located on alpha helixes, beta folds, or fragments with an undefined structure. This was consistent with the results in Figure 7, where repulsion did not correlate with the total helix forming propensity. The second feature is that the repulsive peptides in the case of DKK1 and SFRP1 were located in well-defined secondary structures separated by relatively long unstructured flexible amino acid chains (Fig. 9a and 9b). Since the biologically active secreted form of human TNF-alpha adopts a triangular pyramid shape, mapping was carried out on an experimentally obtained 3D model of the TNF-alpha homotrimer [Eck, M. J. & Sprang, S. R. The Structure of Tumor Necrosis Factor-Alpha at 2.6-a Resolution - Implications for Receptor-Binding. J Biol Chem 264, 17595-17605 (1989*)*] (Fig. 9d). In this case, the part of the protein containing peptides 9 and 10 was cleaved from the secreted form. The TNF-alpha homotrimer was surrounded by fragments that repel cells. In this case, repulsive peptides 11 and 13 with an undefined structure were located at the trimer poles.

### Formation of Cell Patterns

The settlement of cells on alternating patterns of the cell-repellent and cell-adhesive peptides IAMTPPNATEASKPQ and FLCSLFAPVCLDRPI, respectively, were examined over time (Fig. 10). Fig. 10a shows cell deposition after 24 hours of incubation. Peptide patterns in the form of the letters KI were made of squares of 3 sp × 3 sp. The cellular patterns are clearly distinguishable as long as the area of the cell-repellent peptide has a size of 30 µm in diameter (1 sp). To follow the formation of such cellular patterns, the behaviour of cells was examined over time (continuous fluorescence detection). Fig. 10b, 10c, 10d and 10e show the different states of the same chip fragment with cell-repellent peptides at different incubation times. Relatively quickly, in the first minutes after seeding, cells randomly distribute over the entire surface (Fig. 10b) and start to form clusters (Fig. 10c), which adhere to the bottom. After that, the cell clusters leave the area of peptides with cell repulsion (Fig. 10d). The process of cellular clusters leaving the repulsive region can take hours (Fig. 10e). Fig. 10b, 10c, 10d and 10e show an overlay of the fluorescent channels on a bright-field image. The picture shows fluorescence originating from the TOP-GFP reporter, reflecting the activated Wnt pathway, as well as fluorescence resulting from the constitutively expressed mCherry protein.

### Parameters of Cell Patterning on Multifunctional Surfaces (Peptide Chips)

Successful screening of peptides on multifunctional adhesion-modulating surfaces requires control over the rate of formation of cell agglomerates and the intensity of cell migration. Both parameters can be controlled, for example, by cell concentration, temperature and the composition of the cell medium.

In the experiments it has been observed that some of the clusters that were positioned in the area of repulsion (such as at the bottom of the T-area in Fig. 10e) did not completely leave this region. It is assumed that the reason is a sufficiently large part of the agglomerate having been positioned in the region of the attracting peptide resulting in a lack of sufficient migratory capacity of the cells to either break the agglomerate or to move it completely. Such effects can be resolved by prolonging culturing of cells and the incubation time. In the present experiment a prolongation of the culturing time for approximately ten hours was found to be necessary for maximum removal of agglomerates from the area of cell-repellent peptides. This time may have been necessary to change the expression of surface adhesion molecules, which is induced by cancer cells to enter a mode of collective migration [Wolf, K. et al. Multi-step pericellular proteolysis controls the transition from individual to collective cancer cell invasion. Nat Cell Biol 9, 893-U839 (2007].

### Short Peptides' Attraction or Repulsion to Cells

The method of the invention confirmed extreme adhesive properties at the level of short peptides, which is not surprising in the case of adhesion, since protein-protein binding occurs through the selective recognition of short domains by more complex binding grooves.

However, the method of the invention is directed to the identification of cell-repelling peptides, which are considered as useful in modulating cell interactions, as their availability can be controlled through the configuration of the proteins on which they are presented.

Cell patterning was demonstrated on minimal structures on the order of 30 µm, with a spacing of 30 µm between lines (Fig. 10a), which corresponds to a spot density of approximately 28000 peptide candidates per square centimetre. This number of peptides (considering the area of a microscopic slide with more than 20 cm²) allows to implement various cell matrix screening strategies, such as mapping peptides on known proteins (Fig. 2) or substitutional analysis to search for invariant amino acids at the proteomic scale (Fig. 8).

So far no common motifs or elements of strong adhesion or repulsion were identified for the sequences examined herein, which indicates the high selectivity of the observed interactions. This confirms the need for fast, efficient and easy to implement screening methods to allow further investigation in this field.

For example, for the CD44v6 15-mer peptide, only a single mutation was found to be critical to significantly enhance cell repulsion (Fig. 9). Most of the peptides with extreme adhesive properties were found on sequences similar to those that actually exist in proteins. A strong cell-attractive HA epitope is present on haemagglutinin, which is responsible for initial viral attachment to receptors on red blood cells (Fig. 1c). Most of the cell-repellent peptides identified herein with the method of the invention have not been previously reported in the literature.

It has been observed that the KQQ motif, which functions as a switch, can distinguish between the active and inactive states of nitrogen regulatory protein C [Vanatta, D. K., Shukla, D., Lawrenz, M. & Pande, V. S. A network of molecular switches controls the activation of the two-component response regulator NtrC. Nat Commun 6, 7283, doi:10.1038/ncomms8283 (2015*)].* According to the screening carried out herein with the method of the invention, cell-repellent motifs are highly concentrated on secreted proteins, which likely prevents them from non-selectively contacting cells.

With the method of the invention it was possible to show for the first time that cell-repelling peptides are located at the poles of the TNF-alpha homotrimer (Fig. 9d). The results of the screening experiments suggest that initial peptide libraries to screen for peptides with extreme adhesion properties should be designed based on secreted or viral proteins and their mutations.

### Cell Cultivation on Peptide Chips: Potential Applications

The method of the invention allows rapid screening of peptides with optimal properties for cell experiments. Short peptides, being less expensive, can replace proteins and polymers currently used for cellular patterning. Considering the functional diversity of peptides, it is possible to select specific peptides for single-cell studies. Such peptides can be used in complex cell-confining environments, such as two-state systems with a narrow adhesion gap to more sensitively quantify the migration of different cancer cell lines. Invasion by collective migrating carcinomas is known to be characterized by a fine balance between cell-cell and cell-ECM adhesion. Screening of functional peptides controlling such processes can be performed using the method of the invention. In addition, within the framework of the method described herein, it becomes possible to use reporter constructs such as TOP-GFP (Fig. 10b, 10c, 10d and 10e) to detect inhibitors or activators of signalling pathways.

A significant advantage of the screening method of the invention is the ability to detect potential cell-repellent peptides in a fast, efficient and easy to implement way. Identifying peptides that block cytokines or growth factor-induced signalling can be used as a strategy to impair the growth and migration of mutated cells.

### Methods

### Cell Culture

SW620mCherry [Salma M et al. Fam83 induces p53 stabilisation and promotes its activity. Cell Death Differ. 26(10):2125-2138. doi: 10.1038/s41418-019-0281-1. Epub 2019 Jan 28. PMID: 30692643; PMCID: PMC6748130] and HEK293T (ATCC Cat# CRL-3216, RRID: CVCL_0063) cells were grown in DMEM supplemented with 10% foetal bovine serum and 1% penicillin-streptomycin in a humidified incubator at 37°C. All experiments were performed using mycoplasma-free cells.

### Lentiviral Transduction

Stable transduction of the TOP-GFP reporter was achieved using lentiviral particles produced in HEK293T cells. The envelope plasmid (vesicular stomatitis virus G protein, pVSV-G (2.8 µg)), two packaging plasmids (pRSV-REV (2.5 µg) and pMDLg/pRRE (5 µg)) and the plasmid of interest (TOP-GFP (10 µg); Addgene) were transfected into 80-90% confluent HEK293T cells using PromoFectin (PromoKine) according to the manufacturer's protocol. Six hours after transduction, the medium was exchanged. Target (SW620 mCherry) cells were seeded on the same day to reach a confluency of 70% on the day of transduction. Twenty-four hours after the medium exchange, the supernatant containing the produced virus particles was harvested, filtered through a 0.45 µm filter and transferred to the target cells. To increase the number of virus particles, fresh DMEM was applied to virus particle-producing HEK293T cells. Twenty-four hours later, the medium was again harvested and filtered as described above. After transduction, the target cells were selected via fluorescence-activated cell sorting (FACS) (see "Fluorescence-activated cell sorting" section). The cell types were not authenticated.

### Fluorescence-Activated Cell Sorting (FACS)

FACS sorting was performed using a FACSAria TM Fusion Flow Cytometer (BD Biosciences).

For the selection of a monoclonal cell population containing the TOP-GFP reporter, detachment of the cells was performed using Accutase, followed by the collection of the cells in serum-containing medium (DMEM). For the analysis and subsequent sorting, 1x10 7 cells were used and resuspended in 3 ml of FACS buffer (2% FCS; 2 mM EDTA; PBS). Monoclonal sorting was achieved using index sorting in a 96-well plate (1 event per well), followed by reanalysis of the single clones 6 weeks after the initial sorting procedure. Data analysis was performed using FlowJo software (licence number M11c3c353YH92SCS).

### Cell Incubation on Peptide Chips

On the day of incubation, cells were detached using Accutase followed by selection in serum-containing medium (DMEM). Before incubation, the chip was washed with PBS (1% PenStrep) and then clamped the chip in a holder provided for this purpose. To incubate the cells on the chip, 1×10⁷ cells in 3 ml of DMEM (1% PenStrep; 5% FCS; 25 mM HEPES) were transferred onto the chip and incubated for 24 hours either in the incubator or in a live cell imaging incubation chamber. After 24 hours, the plates were either analysed using confocal microscopy or fixed.

### Confocal Scanning Microscopy

Twenty-four hours after seeding, the slides were analysed using an Innoscan 1100 AL confocal fluorescence scanner (Innopsys, Carbonne, France) with resolution 2 µm, PMTGain= 4, excitation wavelength 532 nm, Velocity=35 l/s. Fig. 9 and corresponding movies were made with a Zeiss LSM 800 confocal microscope.

### Confocal Microscopy / Live Cell Imaging

For imaging of the cells, a Zeiss LSM800 confocal microscope was used. Therefore, the cells were seeded as described above. The microscopic slide was then transferred to a live cell incubation chamber (37°C) and observed via continuous fluorescence detection for a period of 24 hours.

Representative pictures and movies were processed using ZEN (Zeiss) and iMovie (Apple) Software.

### Mapping of Peptides on the Proteins

The simulation tool VMD was used for peptide mapping. 3D protein structures were available in the UniProt database and predicted with Al-based software AlphaFold developed by DeepMind (London, UK). SFRP1: AF-Q8N474-F1; DKK1: AF-O94907-F1; TNF-alpha: AF-Q5STB3-F1.

For homotrimer TNF-alpha, the experimental 3D structure was used (PDB DOI:10.2210/pdb1TNF/pdb; Deposition Author(s): Eck, M.J., Sprang, S.R. ; Method: X-ray diffraction, Resolution: 2.60 Å.

**SEQUENCE LIST**

| ***No*** | ***Protein*** | ***Peptide*** |
|---|---|---|
| 1 | SFRP1 | KQQ |
| 2 | SFRP1 | PNATEASKP |
| 3 | SFRP1 | EVK |
| 4 | SFRP1 | AIHKWDKKN |
| 5 | DKK1 | SVLNSNAIK |
| 6 | DKK1 | VSAAP |
| 7 | DKK1 | TLSSKMYHTKGQ |
| 8 | DKK1 | WSKICKPVLKE |
| 9 | TNF-alpha | AEEALPKK |
| 10 | TNF-alpha | VRSSSRTP |
| 11 | TNF-alpha | SDKKPVAHVVANPQAE |
| 12 | TNF-alpha | YQTKV |
| 13 | TNF-alpha | PCQRETPEGAEAKP |
| 14 | SFRP1 | CASEFALRMKIKEVK |
| 15 | SFRP1 | ASEFALRMKIKEVKK |
| 16 | SFRP1 | SEFALRMKIKEVKKE |
| 17 | SFRP1 | EFALRMKIKEVKKEN |
| 18 | SFRP1 | FALRMKIKEVKKENG |
| 19 | SFRP1 | ALRMKIKEVKKENGD |
| 20 | SFRP1 | LRMKIKEVKKENGDK |
| 21 | SFRP1 | RMKIKEVKKENGDKK |
| 22 | SFRP1 | MKIKEVKKENGDKKI |
| 23 | SFRP1 | KIKEVKKENGDKKIV |
| 24 | SFRP1 | IKEVKKENGDKKIVP |
| 25 | SFRP1 | KEVKKENGDKKIVPK |
| 26 | SFRP1 | EVKKENGDKKIVPKK |
| 27 | *DKK1*/*SFRP1* | HPGSAVSASNAIKNL |
| 28 | *DKK1*/*SFRP1* | TPPNATEASKPQGTT |
| 29 | *DKK1*/*SFRP1* | AIKNLPPPTKGQEGS |
| 30 | *DKK1* / *SFRP1* | QETWF |
| 31 | | QNGNQGKN |
| 32 | | IAMTPPNATEASKPQ |
| 33 | | FLCSLFAPVCLDRPI |
| 34 | | KENGDKKIVPKKKKP |
| 35 | | QETWFQNGWQGKN |
| 36 | | QETWFQNGWQGKNP |
| 37 | | KEQWFGNRWHEGYR |

## Claims

1. A method for detecting and/or identifying peptides and/or sequences with cell-repelling properties, which comprises:
(a) preparing or providing a chip which comprises attached to a chip-surface an array of multiple different peptides and/or sequences which are located adjacent to each other on the chip-surface to form a predetermined pattern of peptide- and/or sequence-spots;
(b) placing or providing the chip in a device suitable for receiving and/or culturing cells;
(c) adding cells and allowing the cells in the device to flow freely over the chip surface,
(d) fluorescence detection of the chip surface,
(e) detecting and/or identifying the peptides and/or sequences with cell-repelling properties via fluorescence signals.

2. The method according to claim [1], further comprising a step (i) of microscopic evaluation of the chip surface.

3. The method according to claim [1] or [2], wherein
the peptides are attached to the chip-surface in the form of peptide- and/or sequence spots (pixels) having a size of ≤ 200 µm in diameter, preferably having a size of between 20 µm × 20 µm to 100 µm × 100 µm;
and/or
the distance between the individual adjacent peptide- and/or sequence-spots (pixels) on the chip-surface is ≤ 1 pixel;
and/or
the chip comprises ≥ 1000, preferably ≥ 3000, more preferably ≥ 5000, more preferably ≥ 10000 different peptides and/or sequences attached to the chip-surface;
and/or
the different peptides / sequences are attached to the chip-surface with a spot density of > 20000 peptides / sequences per square centimetre.

4. The method according to any one of claims [1] to [3], wherein the device suitable for receiving and/or culturing cells is an incubation tray, a live cell imaging incubation chamber or a multiwell plate.

5. The method according to any one of claims [1] to [4], which is carried out as high-throughput screening method.

6. The method according to any one of claims [1] to [5], wherein the detection and/or identification of the peptides and/or sequences with cell-repelling properties is carried out by continuous fluorescence detection or by fluorescence detection at a fixed end-point after running the method for a predetermined time or by a combination of both.

7. The method according to any one of claims [1] to [6], wherein the peptides and/or sequences attached to the chip-surface are short-chain peptides with amino acid sequences of between 3 to 20 amino acids.

8. The method according to any one of claims [1] to [6], wherein the peptides attached to the chip-surface are medium- to long-chain peptides with amino acid sequences of more than 20 amino acids.

9. The method according to any one of claims [1] to [8], wherein the peptides attached to the chip-surface can be selected from linear peptides and/or sequences, cyclic peptides and/or sequences and from peptides and/or sequences comprising artificial (non-biogenic) amino acids, and combinations thereof.

10. The method according to any one of claims [1] to [9], wherein the peptides and/or sequences attached to the chip-surface are derived from known protein sequences by fragmentation and/or by substitution of one or more amino acids and/or by permutation of the protein sequences.

11. The method according to any one of claims [1] to [10], wherein
the chip is designed to comprise at least one array of amino acid sequences which are fragments derived from one protein sequence by selecting multiple different fragments with identical chain length of consecutive parts of the protein sequence resulting in an array of amino acid sequence fragments having an overlap with respect to one, two or three amino acids of the protein sequence chain when comparing all fragments;
and/or
the chip is designed to comprise at least one array of substituted amino acid sequences which are fragments derived from one protein sequence wherein in the fragments one amino acid of the originating protein sequence is replaced by one of the remaining nineteen biogenic amino acids.

12. Peptides and/or sequences with cell-repelling properties according to SEQ IDs No.:
| **SEQ ID No.** | **Peptide** |
|---|---|
| 1 | KQQ |
| 2 | PNATEASKP |
| 3 | EVK |
| 4 | AIHKWDKKN |
| 5 | SVLNSNAIK |
| 6 | VSAAP |
| 7 | TLSSKMYHTKGQ |
| 8 | WSKICKPVLKE |
| 9 | AEEALPKK |
| 10 | VRSSSRTP |
| 11 | SDKKPVAHVVANPQAE |
| 12 | YQTKV |
| 13 | PCQRETPEGAEAKP |
| 14 | CASEFALRMKIK**EVK** |
| 15 | ASEFALRMKIK**EVK**K |
| 16 | SEFALRMKIK**EVK**KE |
| 17 | EFALRMKIK**EVK**KEN |
| 18 | FALRMKIK**EVK**KENG |
| 19 | ALRMKIK**EVK**KENGD |
| 20 | LRMKIK**EVK**KENGDK |
| 21 | RMKIK**EVK**KENGDKK |
| 22 | MKIK**EVK**KENGDKKI |
| 23 | KIK**EVK**KENGDKKIV |
| 24 | IK**EVK**KENGDKKIVP |
| 25 | K**EVK**KENGDKKIVPK |
| 26 | **EVK**KENGDKKIVPKK |
| 27 | HPGSAVSASNAIKNL |
| 28 | TPPNATEASKPQGTT |
| 29 | AIKNLPPPTKGQEGS |
| 30 | QETWF |
| 31 | QNGNQGKN |
| 32 | IAMTPPNATEASKPQ |
| 33 | FLCSLFAPVCLDRPI |
| 34 | KENGDKKIVPKKKKP |
| 27 | HPGSAVSASNAIKNL |
| 28 | TPPNATEASKPQGTT |
| 29 | AIKNLPPPTKGQEGS |
| 30 | QETWF |
| 31 | QNGNQGKN |
| 32 | IAMTPPNATEASKPQ |
| 33 | FLCSLFAPVCLDRPI |
| 34 | KENGDKKIVPKKKKP |
and homologues thereof having at least 70% identity, preferably at least 80% identity, more preferably at least 85% identity, even more preferably at least 90% identity, even more preferably at least 95% identity, and the sequences of SEQ ID 1 to 34, wherein one amino acid is replaced by one of the remaining nineteen biogenic amino acids.

13. Use of the peptides and/or sequences according to SEQ IDs No.:
| **SEQ ID No** | **Peptide** |
|---|---|
| 1 | KQQ |
| 2 | PNATEASKP |
| 3 | EVK |
| 4 | AIHKWDKKN |
| 5 | SVLNSNAIK |
| 6 | VSAAP |
| 7 | TLSSKMYHTKGQ |
| 8 | WSKICKPVLKE |
| 9 | AEEALPKK |
| 10 | VRSSSRTP |
| 11 | SDKKPVAHVVANPQAE |
| 12 | YQTKV |
| 13 | PCQRETPEGAEAKP |
| 14 | CASEFALRMKIK**EVK** |
| 15 | ASEFALRMKIK**EVK**K |
| 16 | SEFALRMKIK**EVK**KE |
| 17 | EFALRMKIK**EVK**KEN |
| 18 | FALRMKIK**EVK**KENG |
| 19 | ALRMKIK**EVK**KENGD |
| 20 | LRMKIK**EVK**KENGDK |
| 21 | RMKIK**EVK**KENGDKK |
| 22 | MKIK**EVK**KENGDKKI |
| 23 | KIK**EVK**KENGDKKIV |
| 24 | IK**EVK**KENGDKKIVP |
| 25 | K**EVK**KENGDKKIVPK |
| 26 | **EVK**KENGDKKIVPKK |
| 27 | HPGSAVSASNAIKNL |
| 28 | TPPNATEASKPQGTT |
| 29 | AIKNLPPPTKGQEGS |
| 30 | QETWF |
| 31 | QNGNQGKN |
| 32 | IAMTPPNATEASKPQ |
| 33 | FLCSLFAPVCLDRPI |
| 34 | KENGDKKIVPKKKKP |
| 27 | HPGSAVSASNAIKNL |
| 28 | TPPNATEASKPQGTT |
| 29 | AIKNLPPPTKGQEGS |
| 30 | QETWF |
| 31 | QNGNQGKN |
| 32 | IAMTPPNATEASKPQ |
| 33 | FLCSLFAPVCLDRPI |
| 34 | KENGDKKIVPKKKKP |
| 35 | QETWFQNGWQGKN |
| 36 | QETWFQNGWQGKNP |
| 37 | KEQWFGNRWHEGYR |
and homologues thereof having at least 70% identity, preferably at least 80% identity, more preferably at least 85% identity, even more preferably at least 90% identity, even more preferably at least 95% identity, and the sequences of SEQ ID 1 to 37, wherein one amino acid is replaced by one of the remaining nineteen biogenic amino acids, as cell-repelling peptides and/or sequences for the modification of surfaces.

14. The use according to claim [13] for the modification of surfaces in the medical, biomedical, biochemical field, tissue engineering.

15. The use according to claim [13] or [14] for the modification of surfaces of medical or surgical devices, medical, biomedical, biochemical, diagnostic or tissue engineering equipment.
